# NOUVEAU FASCICULE DE BREVET EUROPEEN

(11) **EP 3 480 509 B2**
(45) Date de publication et mention de la décision concernant l'opposition: **21.05.2025**
(45) Mention de la délivrance du brevet: 13.05.2020
(21) Numéro de dépôt: 18205019.5
(22) Date de dépôt: 16.02.2015
(51) Int. Cl.: F17C 13/06, A61M 16/10

(54) **UTILISATION D'UNE BOUTEILLE DE GAZ AVEC CAPOTAGE DE PROTECTION AVEC SYSTÈME D'ACCROCHAGE PIVOTANT**
NUTZUNG EINER GASFLASCHE MIT SCHUTZHAUBE MIT SCHWENKBAREM EINRASTSYSTEM
USE OF A GAS CYLINDER WITH A PROTECTIVE HOOD WITH PIVOTING ATTACHMENT SYSTEM

(30) Priorité: 12.03.2014 FR 1452039
(43) Date de publication de la demande: 08.05.2019
(62) Demande divisionnaire de: 15305224.6
(73) Titulaire: L'AIR LIQUIDE, SOCIETE ANONYME POUR L'ETUDE ET L'EXPLOITATION DES PROCEDES GEORGES CLAUDE, 75007 Paris (FR)
(72) Inventeur: TARANTELLO, Chiara, 92400 Courbevoie (FR); LIGONESCHE, Renaud, 78350 Jouy-en-Josas (FR); FRENAL, Antoine, 78350 Jouy-en-Josas (FR); GRAVIERE, Vincent, 94200 Ivry sûr Seine (FR); TREVISAN, Adrien, 94200 Ivry sûr Seine (FR); VIGNEROL, Samuel, 78350 Jouy-en-Josas (FR)
(74) Mandataire: Air Liquide

(56) Documents cités:
- EP-A1- 0 629 812
- EP-A1- 2 330 335
- EP-A1- 2 586 481
- WO-A1-2013/001190
- WO-A1-2015/036663
- WO-A2-2015/132092
- DE-A1- 10 057 469
- FR-A1- 2 918 152
- GB-A- 2 246 596
- US-A- 5 429 152

## Description

La présente invention concerne l'utilisation d'un récipient de gaz équipé d'un bloc robinet, d'un capotage de protection, encore appelé chapeau, telle une bouteille de gaz, en particulier de gaz médical, et d'un dispositif d'accrochage sur lits, brancards, fauteuils ou autres, venant épouser les contours extérieurs du corps de capotage lorsqu'il est replié dans sa position de repos.

Les gaz industriels et médicaux sont couramment conditionnés dans des récipients de gaz, typiquement des bouteilles de gaz, équipés d'un bloc robinet, avec ou sans détendeur intégré, à savoir un robinet simple de type ouvert/fermé ou un robinet à détendeur intégré, encore appelé RDI, permettant de contrôler débit et pression du gaz délivré.

Afin de protéger ce bloc robinet, il est courant agencer au niveau du col de la bouteille de gaz et autour dudit bloc robinet, un capotage de protection formant coque protectrice autour dudit bloc robinet. Un tel capotage est couramment appelé « chapeau ».

Par ailleurs, pour permettre d'accrocher l'ensemble bouteille/chapeau/robinet à un support, tel un barreau de lit d'hôpital, un brancard, une tringle ou support d'accrochage d'un véhicule d'urgence, par exemple une unité de SAMU ou analogue, il est usuel d'aménager sur le chapeau, un système d'accrochage fixe.

En fait, il existe deux types de systèmes d'accrochage, à savoir ceux fixes et ceux mobiles.

A titre d'exemple de système d'accrochage fixe, on peut citer les encoches, crochets ou analogues aménagés portés par le capotage, soit lors de la fabrication du corps du capotage lui-même et qui en font intégralement partie, soit qui sont fixés au corps de capotage après coup.

A titre d'exemple de système d'accrochage mobile, on peut citer les supports pivotants ou translatifs venant se fixer à la face arrière ou à une face latérale du capotage.

Des capotages pour bouteilles de gaz à système d'accrochage fixe ou mobile sont décrits par les documents EP-A-629812, DE-A-10057469, US-A-2004/020793 et EP-A-2586481.

Toutefois, ces différents capotages et systèmes d'accrochage existants présentent des inconvénients.

Ainsi, beaucoup de systèmes d'accrochage s'étendent au-delà du corps de la bouteille, même lorsqu'ils sont repliés dans leur position de repos, ce qui conduit à augmenter l'encombrement important de l'ensemble bouteille/capotage.

Or, augmenter l'encombrement de l'ensemble engendre, de façon aisément compréhensible, des problèmes de stockage des bouteilles ainsi équipées, en particulier de rangement dans les racks ou autres paniers servant à leur transport groupé.

De plus, le fait que beaucoup dépassent de systèmes d'accrochage pivotants du diamètre général de la bouteille, même repliés, est une source d'accidents pour les utilisateurs dont les vêtements par exemple peuvent s'accrocher par inadvertance au système et engendrer une chute sur le sol de la bouteille, avec un risque de blessure pour les personnels à proximité.

Ainsi, le document EP-A-2586481 enseigne un système d'accrochage pivotant pour chapeau de bouteille de gaz. Ce système est formé de deux bras pivotants qui sont reliés l'un à l'autre par une tige rigide. En position repliée, la tige vient buter sur le corps du chapeau en restant tangentielle à celui-ci, alors que les deux bras viennent se placer de part et d'autre du haut du corps de la bouteille. Un tel système présente donc les inconvénients susmentionnés.

D'autres sont d'architecture compliquée et/ou sont peu pratiques à manipuler.

Il existe aussi un capotage possédant un logement spécifique dans lequel vient s'insérer un bras pivotant. Si cela permet d'éviter que le bras ne déborde du corps du capotage, lorsqu'il est replié, cela a aussi comme inconvénient majeur de rendre le nettoyage les parties intérieures du capotage et du dispositif d'accrochage plus compliqué et d'engendrer une accumulation inévitable de poussières et autres saletés au fil du temps dans ces régions du capotage, c'est-à-dire un état de propreté peu compatible avec une utilisation en domaine hospitalier. En outre, la manipulation de ce bras pivotant n'est pas forcément aisée pour l'utilisateur qui peut se coincer les doigts lors de son déploiement et de l'accrochage de l'ensemble à un support, tel un barreau de lit d'hôpital.

On connait aussi :
- WO2015/036663 (non publié) et WO2015/132092 (non publié) qui enseignent des capotages de protection pour récipient de gaz équipés chacun d'un bloc robinet et d'un corps de capotage formant coque protectrice autour du bloc robinet, et un dispositif d'accrochage pivotant comprenant deux bras.
- WO-A-2013/001190 enseigne un capotage pour récipient de gaz muni de poignées de portage et d'un crochet d'attache ou suspension permettant de suspendre le récipient par son chapeau à un barreau de lit. Dans un mode de réalisation, les bras du crochet d'attache sont coulissants dans le corps du capotage, alors que dans un autre mode de réalisation, le crochet d'attache pivote mais ses bras ne sont jamais positionnés de part et d'autre du corps du récipient.
- FR-A-2918152 propose un capotage pour récipient de gaz muni d'une poignée de portage pivotante configurée pour venir s'accrocher à un barreau tubulaire, lorsque la poignée est repliée, c'est-à-dire non pivotée, en formant un logement entre une zone de préhension de la poignée pivotante et le corps du récipient.

Au vu de cela, le problème qui se pose est de proposer une utilisation améliorée d'un récipient de gaz équipé d'un bloc robinet et d'un capotage de protection ou chapeau pour bouteille de gaz comprenant un dispositif d'accrochage ne présentant pas les inconvénients susmentionnés.

La solution de l'invention concerne l'utilisation d'un récipient de gaz équipé d'un capotage de protection ou « chapeau », en particulier une bouteille de gaz, et d'un bloc robinet, avec ou sans détendeur intégré, ledit capotage de protection comprenant :
- un corps de capotage formant coque protectrice délimitant un volume interne conçu pour recevoir tout ou partie du bloc robinet, et
- un dispositif d'accrochage pivotant entre plusieurs positions angulaires comprenant :
   . une position de repos dans laquelle le dispositif d'accrochage est totalement replié, c'est-à-dire en contact ou quasi-contact avec le corps du capotage, et
   . une position d'accrochage dans laquelle le dispositif d'accrochage est totalement déplié, c'est-à-dire complètement sorti de manière à permettre l'accroche du capotage à un support, tel un barreau de lit ou analogue,
le dispositif d'accrochage comprenant une structure tridimensionnelle comprenant deux bras portant chacun une encoche de fixation à un support, ledit structure tridimensionnelle comprenant une face intérieure conformée pour venir épouser le profil extérieur du corps de capotage, lorsque le dispositif d'accrochage est en position de repos, c'est-à-dire qu'il est complètement replié et positionné autour du capotage, conformément à la revendication 1.

Selon le cas, le capotage de protection utilisé selon l'invention peut comprendre l'une ou plusieurs des caractéristiques techniques suivantes :
- le dispositif d'accrochage est fixé de manière pivotante autour d'un axe AA situé sur la face arrière du corps du capotage.
- la face arrière et/ou les faces latérales du corps de capotage est/sont conformées pour présenter un profil extérieur complémentaire de la face intérieure de la structure tridimensionnelle.
- la face arrière et/ou les faces latérales du corps de capotage présentent au moins une empreinte réalisée dans la coque externe du corps dans laquelle vient se loger au moins une partie de la structure tridimensionnelle en épousant le profil extérieur de ladite au moins une empreinte.
- la structure tridimensionnelle comprend au moins une face intérieure incurvée.
- la structure tridimensionnelle a une forme générale incurvée.
- il comprend un système de blocage permettant de bloquer et maintenir le dispositif d'accrochage en position de repos.
- le dispositif d'accrochage pivote entre la position de repos et la position d'accrochage d'un angle (α) d'au moins 110°.
- il comprend au moins un montant-support solidaire du corps de capotage et portant une poignée de portage.
- le corps du capotage et la structure tridimensionnelle sont formés de matériau polymère. Typiquement, le matériau de type polymère est un matériau plastique, tel que PVC, PE, PET, PP, PMMA, PU, PA...
- les deux bras sont reliés l'un à l'autre par au moins un élément de jonction, de préférence les deux bras et ledit au moins un élément de jonction sont formés d'une seule pièce, notamment par moulage.
- il comprend un système de blocage permettant de bloquer et maintenir le dispositif d'accrochage en position de repos.

Selon le cas, le récipient de gaz utilisé selon l'invention peut comprendre l'une ou plusieurs des caractéristiques techniques suivantes :
- il est choisi parmi les bouteilles de gaz.
- il est une bouteille de gaz ayant une taille comprise entre 10 et 150 cm.
- il est une bouteille de gaz contenant de 0,5 à 20 litres (contenance en équivalent eau).
- il est une bouteille de gaz de corps cylindrique creux.
- il est une bouteille de gaz comprenant un col portant un orifice de sortie du gaz.
- le bloc robinet est de type à détendeur intégré, c'est-à-dire un RDI.
- la bouteille est en acier, en un alliage d'aluminium ou en matériau composite.
- la bouteille contient un gaz à une pression allant jusqu'à 350 bar environ.

L'invention va maintenant être mieux comprise grâce à la description détaillée suivante, faite à titre illustratif mais non limitatif, en référence aux figures annexées parmi lesquelles :
- la Figure 1 représente une vue de la face avant d'un capotage de protection équipant une bouteille de gaz utilisée selon l'invention,
- la Figure 2 représente une vue de la face arrière du capotage de la Figure 1,
- la Figure 3 est une vue de ¾ arrière du capotage de la Figure 1, et
- la Figure 4 représente un ensemble formé d'une bouteille de gaz équipée du capotage de la Figure 1 vu en position couchée sur une surface.

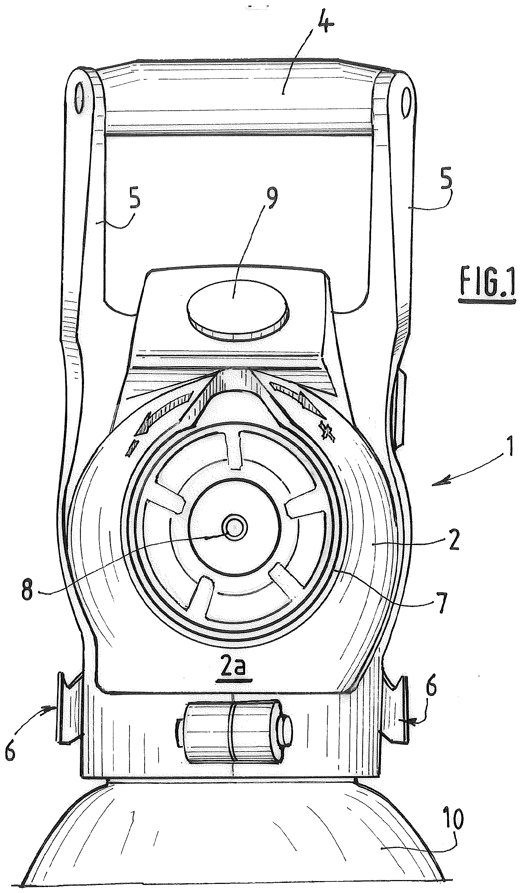

Les Figures 1 à 4 présentent un mode de réalisation d'un capotage de protection 1 rigide, couramment appelé « chapeau », agencé autour d'un bloc robinet, à savoir un robinet avec ou sans détendeur intégré, lui-même fixé sur le col d'une bouteille de gaz 10.

La bouteille de gaz 10 a typiquement une taille entre 10 et 150 cm, et une contenance de 0,5 à 20 litres (en équivalent eau).

Le capotage de protection 1 sert à protéger le bloc robinet contre les chocs, que le dit bloc robinet soit du type avec détendeur intégré ou RDI, ou du type sans détendeur intégré.

A ce titre, le capotage de protection 1 comprend un corps 2 de capotage formant une coque protectrice autour d'un volume interne dimensionné pour recevoir le bloc robinet, une poignée de portage 4 conçue pour être prise en main par un opérateur, ladite poignée de portage 4 étant formée d'un matériau rigide, et portée par un ou plusieurs montants-supports 5 reliant mécaniquement le corps 2 de capotage à la poignée de portage 4, c'est-à-dire que le ou les montants-supports 5 sont solidarisés à la poignée de portage 4 de manière à permettre à un utilisateur de transporter facilement l'ensemble chapeau/robinet/bouteille.

Le corps 2 du capotage 1 est typiquement en un matériau de type polymère et/ou métal, préférentiellement en matériau plastique, tel que PVC, PE, PET, PP, PMMA, PU, PA... De même, les montants-supports 5 peuvent être formés d'un matériau plastique, comme le corps du capotage 1, mais aussi en alliage d'aluminium ou tout autre matériau métallique.

Le capotage de protection 1 présente par ailleurs des ouvertures donnant accès au bloc robinet situé dans le volume interne du corps 2 de capotage 1, en particulier aux raccords, volant rotatif...

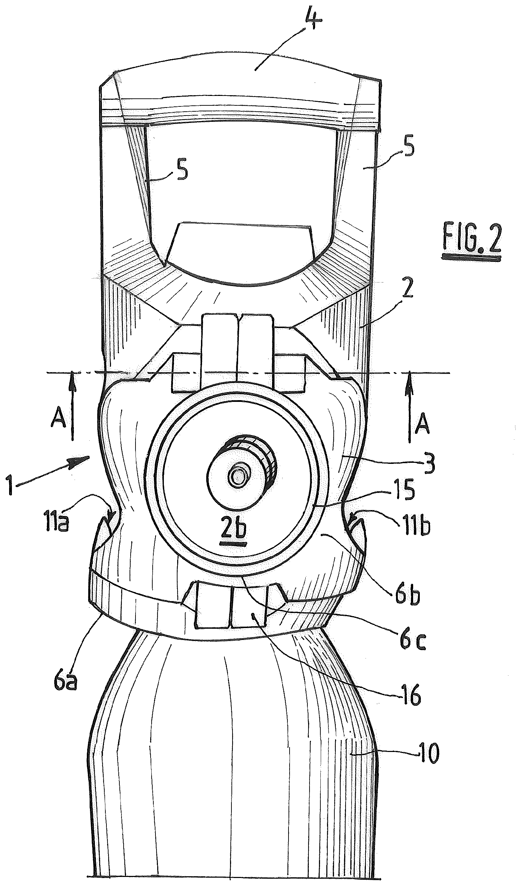

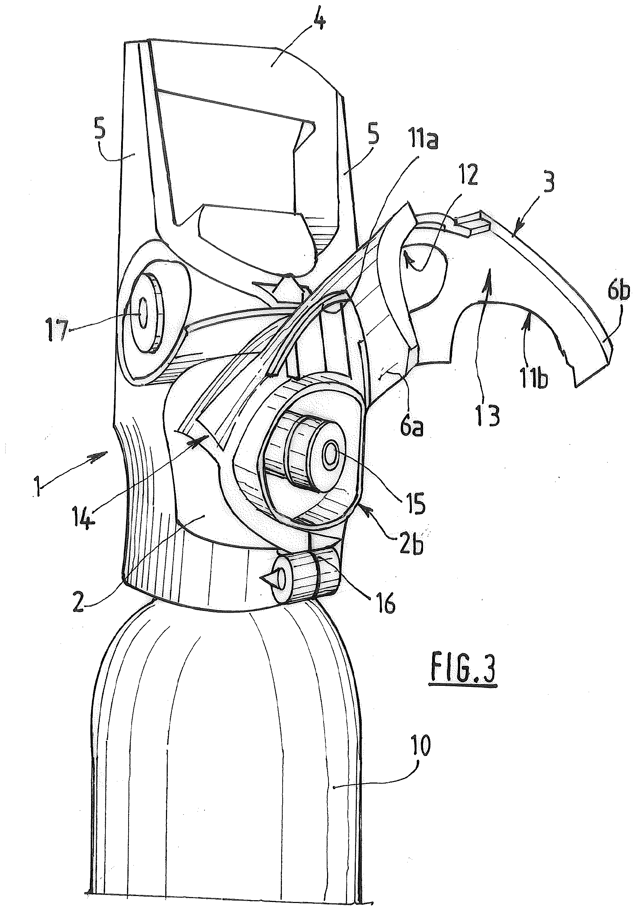

En effet, le bloc robinet, typiquement un robinet de type RDI, comprend, comme illustré en Figure 3, un volant rotatif 7, situé en face avant 2a, manipulable par un utilisateur pour contrôler le débit de gaz, un raccord de sortie de gaz 8 pour soutirer le gaz stocké dans la bouteille 10, un manomètre 9 pour contrôler la pression du gaz, un raccord de remplissage 15 (Fig.2 et 3), situé en face arrière 2b, servant à introduire du gaz dans la bouteille 10 lorsque celle-ci est vide, et un raccord de sortie de gaz sous pression 17

L'assemblage du capotage de protection 1 et sa fixation autour du bloc de robinet porté par la bouteille de gaz 10, se fait par vissage au moyen d'éléments de fixation, à savoir des vis ou analogues.

Afin de permettre l'accroche ou l'arrimage de l'ensemble bouteille/bloc robinet/capotage à un support, tel un barreau de lit d'hôpital ou de brancard, le capotage de protection 1 comprend, du côté de sa face arrière 2b, un dispositif d'accrochage 3 pivotant autour d'un axe AA, entre une position totalement repliée, dite « de repos », c'est-à-dire la position adoptée par le dispositif d'accrochage 3 lorsqu'il est rangé et en contact ou quasi-contact du corps 2 du capotage 1, et une position totalement dépliée dite « d'accrochage », c'est-à-dire la position adoptée par le dispositif d'accrochage 3 lorsqu'il est complètement sorti et peut être accroché à un support, tel un barreau de lit ou analogue.

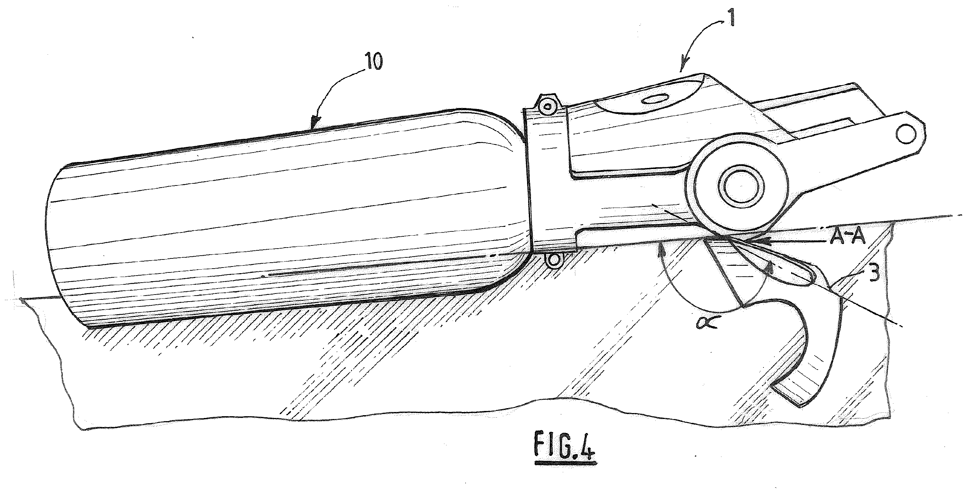

Ainsi, la Figure 2 montre le dispositif d'accrochage 3 en position de repos, c'est-à-dire replié, alors que les Figures 3 et 4 montrent le dispositif d'accrochage 3 en position d'accrochage, c'est-à-dire totalement déplié.

Ces deux positions constituent donc les positions angulaires extrêmes que peut adopter le dispositif d'accrochage 3 lors de son pivotement autour de l'axe AA.

Typiquement, l'angle α de pivotement entre ces deux positions angulaires extrêmes est inférieur ou égale à 170° et supérieur à 0°, préférentiellement supérieur à 90°, par exemple un angle de l'ordre de 150°.

Afin de résoudre certains des problèmes posés par les dispositifs d'accrochage connus, dans le cadre de la présente invention, on veille à une parfaite intégration du dispositif d'accrochage 3 autour du corps 2 du capotage de protection 1.

Pour ce faire, le dispositif d'accrochage 3 selon l'invention forme une structure tridimensionnelle incurvée 3, 6a, 6b, 11a, 11b venant épouser les contours du corps 2 de capotage, lorsque le dispositif d'accrochage 3 est en position de repos, c'est-à-dire replié comme illustré en figure 2.

Plus précisément, la structure tridimensionnelle incurvée 3, 6a, 6b, 11a, 11b comprend deux bras 6a, 6b, à savoir un bras droit 6a et un bras gauche 6b, venant prendre le corps 2 de capotage en sandwich, lorsque le dispositif d'accrochage 3 est en position de repos.

Chacun des bras 6a, 6b porte une encoche de fixation 11a, 11b servant à l'accrochage de l'ensemble à un support, tel un barreau de lit d'hôpital ou analogue. Chaque encoche de fixation 11a, 11b a une forme arquée, par exemple conformée en arc de cercle, de manière à épouser au mieux la forme d'un barreau tubulaire de lit, de brancard, de fauteuil roulant ou autre.

En d'autres termes, les encoches de fixation 11a, 11b des bras 6a, 6b sont conformées et dimensionnées pour permettre un arrimage d'un récipient de gaz équipé d'un capotage selon l'invention, à un barreau de lit hospitalier ou analogue, c'est-à-dire à une structure tubulaire.

La structure tridimensionnelle 3, 6a, 6b, 11a, 11b comprend, quant à elle, une face intérieure 13 conformée pour venir épouser le profil extérieur 14, c'est-à-dire la surface ou contour externe, du corps 2 de capotage 1 de manière à minimiser l'encombrement général du capotage 1, lorsque le dispositif d'accrochage 3 est replié comme sur la figure 2.

D'une façon générale selon l'invention, la face intérieure 13 de la structure tridimensionnelle, c'est-à-dire la surface ou paroi 13 de la structure tridimensionnelle 3, 6a, 6b, 11a, 11b faisant face au capotage, en particulier celle des deux bras 6a, 6b, est conformée de manière à venir épouser la forme ou le profil externe 14 du corps 2 de capotage 1 de sorte de minimiser l'encombrement global de la structure tridimensionnelle 3, 6a, 6b, 11a, 11b, lorsqu'elle est repliée, et donc éviter tout ou partie des inconvénients des capotages de l'art antérieur.

Ainsi, comme visible sur la Figure 3, la face 13 vient parfaitement épouser le contour extérieur de la face arrière 2b du capotage 1 en venant s'insérer dans l'empreinte 14 aménagée dans le corps 2 du capotage 1, laquelle empreinte 14 a une forme complémentaire de celle de la structure tridimensionnelle 3, 6a, 6b, 11a, 11b, lorsque la structure tridimensionnelle 3, 6a, 6b, 11a, 11b est dans sa position de repos ou totalement repliée, comme montré en Figure 2.

En d'autres termes, afin d'accroître l'intégration de la structure tridimensionnelle 3, 6a, 6b, 11a, 11b dans le corps 2 du capotage 1, on prévoit d'aménager sur le contour du corps 2 de capotage 1, au moins une empreinte 14 spécifique qui soit adaptée et conformée pour recevoir tout ou partie de la structure tridimensionnelle 3, 6a, 6b, 11a, 11b qui vient alors s'y loger en position repliée, c'est-à-dire de repos.

Ainsi, lorsque la structure tridimensionnelle 3, 6a, 6b, 11a, 11b est repliée, elle ne fait pas ou quasiment pas saillie extérieurement et n'augmente pas l'encombrement global du capotage 1.

La structure tridimensionnelle 3, 6a, 6b, 11a, 11b, outre l'accroche à un support tel un barreau de lit d'hôpital ou analogue, a une autre fonction qui est de stabiliser la bouteille 10 en position horizontale pour éviter qu'elle ne roule sur le sol ou sur toute autre surface. Pour ce faire, la structure tridimensionnelle 3, 6a, 6b, 11a, 11b, une fois totalement dépliée donc dans sa position d'accroche, peut servir de béquilles à la bouteille 10 lorsqu'elle est couchée à l'horizontal, comme illustré en Figure 4.

Par ailleurs, dans une variante de réalisation, la structure tridimensionnelle 3, 6a, 6b, 11a, 11b est conformée de manière à permettre une protection du raccord de remplissage 15 de la poussière, des intempéries, des mauvais usages...

A cette fin, l'ouverture ou espacement 12 entre les deux bras 6a, 6b peut être comblé et former un couvercle protecteur venant recouvrir le raccord de remplissage 15 situé sur la face arrière 2b du corps 2, lorsque la structure tridimensionnelle 3, 6a, 6b, 11a, 11b est en position de repos, c'est-à-dire repliée contre le corps 2 du capotage 1.

L'invention est particulièrement bien adaptée à une utilisation dans le domaine de la santé, pour distribuer un gaz ou mélange gazeux médical.

## Revendications

1. Utilisation d'un récipient de gaz (10) équipé d'un bloc robinet et d'un capotage (1) de protection agencé autour d'au moins une partie dudit bloc robinet pour distribuer un gaz ou mélange gazeux, ledit capotage de protection (1), comprenant un corps de capotage (2) formant coque protectrice délimitant un volume interne conçu pour recevoir tout ou partie du bloc robinet, et un dispositif d'accrochage (3) pivotant entre plusieurs positions angulaires comprenant une position de repos dans laquelle le dispositif d'accrochage (3) est totalement replié et une position d'accrochage dans laquelle le dispositif d'accrochage (3) est totalement déplié, ledit dispositif d'accrochage (3) comprenant en outre une structure tridimensionnelle (3, 6a, 6b, 11a, 11b) comprenant deux bras (6a, 6b) portant chacun une encoche (11a, 11b) de fixation à un support, ladite structure tridimensionnelle (3, 6a, 6b, 11a, 11b) comprenant une face intérieure (13) conformée pour venir épouser le profil extérieur (14) du corps (2) de capotage (1), lorsque le dispositif d'accrochage (3) est en position de repos,
ladite utilisation étant **caractérisée en ce que** le gaz ou mélange gazeux est choisi parmi l'oxygène, l'air et les mélanges N₂O/O₂, He/O₂ et NO/azote,
et **en ce que** :
- le dispositif d'accrochage (3) du capotage (1) pivote entre la position de repos et la position d'accrochage d'un angle (α) compris entre 90° et 170°,
- les deux bras (6a, 6b) viennent prendre le corps (2) de capotage en sandwich, lorsque le dispositif d'accrochage (3) est en position de repos, et
- les encoches de fixation (11a, 11b) desdits deux bras (6a, 6b) ont une forme arquée et sont dimensionnées pour permettre un arrimage à une structure tubulaire.

2. Utilisation selon la revendication précédente, **caractérisée en ce que** le dispositif d'accrochage (3) du capotage (1) est fixé de manière pivotante autour d'un axe AA situé sur la face arrière (2b) du corps (2) du capotage (1).

3. Utilisation selon l'une des revendications précédentes, **caractérisée en ce que** la face arrière (2b) et/ou les faces latérales du corps (2) de capotage (1) sont conformées pour présenter un profil extérieur complémentaire de la face intérieure (13) de la structure tridimensionnelle (3, 6a, 6b, 11a, 11b).

4. Utilisation selon l'une des revendications précédentes, **caractérisée en ce que** la face arrière (2b) et/ou les faces latérales du corps (2) de capotage (1) présentent au moins une empreinte (14) réalisée dans la coque externe du corps (2) dans laquelle vient se loger au moins une partie de la structure tridimensionnelle (3, 6a, 6b, 11a, 11b) en épousant le profil extérieur de ladite au moins une empreinte (14).

5. Utilisation selon l'une des revendications précédentes, **caractérisée en ce que** la structure tridimensionnelle (3, 6a, 6b, 11a, 11b) comprend au moins une face intérieure (13) incurvée.

6. Utilisation selon l'une des revendications précédentes, **caractérisée en ce que** le dispositif d'accrochage (3) du capotage (1) pivote entre la position de repos et la position d'accrochage d'un angle (α) d'au moins 110°.

7. Utilisation selon l'une des revendications précédentes, **caractérisée en ce que** le capotage (1) comprend au moins un montant-support (5) solidaire du corps de capotage (2) et portant une poignée de portage (4).

8. Utilisation selon l'une des revendications précédentes, **caractérisée en ce que** le récipient de gaz (10) est choisi parmi les bouteilles de gaz.

9. Utilisation selon l'une des revendications précédentes, **caractérisée en ce que** le récipient de gaz (10) est une bouteille de gaz ayant une taille comprise entre 10 et 150 cm, et contenant de 0,5 à 20 litres (contenance en équivalent eau).

## Patentansprüche

1. Verwendung eines Gasbehälters (10), der mit einem Schließhahnblock und einer Schutzhaube (1) ausgestattet ist, die um mindestens einen Teil des Schließhahnblocks herum angeordnet ist, um ein Gas oder Gasgemisch zu verteilen, wobei die Schutzhaube (1) einen Haubenkörper (2) umfasst, der eine Schutzhülle bildet, die ein Innenvolumen begrenzt, das gestaltet ist, um den Schließhahnblock ganz oder teilweise aufzunehmen, und eine Einrastvorrichtung (3), die zwischen mehreren Winkellagen, umfassend eine Ruheposition, in der die Einrastvorrichtung (3) vollständig eingeklappt ist, und eine Einrastposition, in der die Einrastvorrichtung (3) vollständig ausgeklappt ist, schwenkbar ist, wobei die Einrastvorrichtung (3) weiter eine dreidimensionale Struktur (3, 6a, 6b, 11a, 11b) umfasst, die zwei Arme (6a, 6b) umfasst, die jeweils eine Kerbe (11a, 11b) zur Befestigung an einer Halterung tragen, wobei die dreidimensionale Struktur (3, 6a, 6b, 11a, 11b) eine Innenseite (13) umfasst, die so geformt ist, dass sie sich dem Außenprofil (14) des Körpers (2) der Haube (1) anpasst, wenn die Einrastvorrichtung (3) in der Ruheposition ist, und
wobei die Verwendung **dadurch gekennzeichnet ist, dass** das Gas oder Gasgemisch ausgewählt ist aus Sauerstoff, Luft, und den Gemischen N₂O/O₂, He/O₂ und NO/Stickstoff,
und dass:
- die Einrastvorrichtung (3) der Haube (1) zwischen der Ruheposition und der Einrastposition um einen Winkel (α) zwischen 90° und 170° schwenkt,
- die beiden Arme (6a, 6b) den Haubenkörper (2) sandwich-artig umschließen, wenn die Einrastvorrichtung (3) in der Ruheposition ist, und
- die Befestigungskerben (11a, 11b) der beiden Arme (6a, 6b) eine geschweifte Form haben und so bemessen sind, dass ein Verzurren mit einer rohrförmigen Struktur gestattet ist.

2. Verwendung nach dem vorstehenden Anspruch, **dadurch gekennzeichnet, dass** die die Einrastvorrichtung (3) der Haube (1) um eine AA-Achse auf schwenkbare Weise befestigt ist, die sich auf der Rückseite (2b) des Körpers (2) der Haube (1) befindet.

3. Verwendung nach einem der vorstehenden Ansprüche, **dadurch gekennzeichnet, dass** die Rückseite (2b) und/oder die lateralen Seiten des Körpers (2) der Haube (1) so geformt sind, um ein Außenprofil aufzuweisen, das mit der Innenseite (13) der dreidimensionalen Struktur (3, 6a, 6b, 11a, 11b) komplementär ist.

4. Verwendung nach einem der vorstehenden Ansprüche, **dadurch gekennzeichnet, dass** die Rückseite (2b) und/oder die lateralen Seiten des Körpers (2) der Haube (1) mindestens eine Prägung (14) in der Außenhülle des Körpers (2) aufweisen, in der mindestens ein Teil der dreidimensionalen Struktur (3, 6a, 6b, 11a, 11b) durch Anpassen an das Außenprofil der mindestens einen Prägung (14) untergebracht ist.

5. Verwendung nach einem der vorstehenden Ansprüche, **dadurch gekennzeichnet, dass** die dreidimensionale Struktur (3, 6a, 6b, 11a, 11b) mindestens eine gekrümmte Innenseite (13) umfasst.

6. Verwendung nach einem der vorhergehenden Ansprüche, **dadurch gekennzeichnet, dass** die Einrastvorrichtung (3) der Haube (1) zwischen der Ruheposition und der Einrastposition um einen Winkel (α) von mindestens 110° schwenkt.

7. Verwendung nach einem der vorstehenden Ansprüche, **dadurch gekennzeichnet, dass** die Haube (1) mindestens eine Haltestütze (5) umfasst, die fest mit dem Haubenkörper (2) verbunden ist und einen Tragegriff (4) trägt.

8. Verwendung nach einem der vorstehenden Ansprüche, **dadurch gekennzeichnet, dass** der Gasbehälter (10) aus den Gasflaschen ausgewählt wird.

9. Verwendung nach einem der vorstehenden Ansprüche, **dadurch gekennzeichnet, dass** der Gasbehälter (10) eine Gasflasche ist, die eine Größe zwischen 10 und 150 cm aufweist, und die 0,5 bis 20 Liter (Wasseräquivalentkapazität) enthält.

## Claims

1. Use of a gas container (10) equipped with a tap unit and a protective cowling (1) arranged around at least one portion of said tap unit to dispense a gas or gaseous mixture, said protective cowling (1), comprising a cowling body (2) forming a protective shell delimiting an inner volume designed to receive all or part of the tap unit, and a hanging device (3) pivoting between several angular positions comprising a rest position, wherein the hanging device (3) is totally folded and a hanging position, wherein the hanging device (3) is totally unfolded, said hanging device (3) further comprising a three-dimensional structure (3, 6a, 6b, 11a, 11b) comprising two arms (6a, 6b) each carrying a support-fixing notch (11a, 11b), said three-dimensional structure (3, 6a, 6b, 11a, 11b) comprising an inner face (13) shaped to mould the outer profile (14) of the cowling (1) body (2), when the hanging device (3) is in the rest position,
said use being **characterized in that** the gas or gaseous mixture is selected from among oxygen, air and N₂O/O₂, He/O₂ and NO/nitrogen mixtures,
and **in that**:
- the hanging device (3) of the cowling (1) pivots between the rest position and the hanging position by an angle (α) between 90° and 170°,
- the two arms (6a, 6b) sandwich the cowling body (2), when the hanging device (3) is in the rest position, and
- the fixing notches (11a, 11b) of said two arms (6a, 6b) are arch-shaped and dimensioned to enable fastening to a tubular structure.

2. Use according to the preceding claim, **characterised in that** the hanging device (3) of the cowling (1) is fixed in a pivoting manner about an axis AA situated on the rear face (2b) of the body (2) of the cowling (1).

3. Use according to one of the preceding claims, **characterised in that** the rear face (2b) and/or the side faces of the cowling (1) body (2) are shaped to have a complementary outer profile of the inner face (13) of the three-dimensional structure (3, 6a, 6b, 11a, 11b).

4. Use according to one of the preceding claims, **characterised in that** the rear face (2b) and/or the side faces of the cowling (1) body (2) having at least one footprint (14) made in the outer shell of the body (2), wherein at least one part of the three-dimensional structure (3, 6a, 6b, 11a, 11b) is housed by moulding the outer profile of said at least one footprint (14).

5. Use according to one of the preceding claims, **characterised in that** the three-dimensional structure (3, 6a, 6b, 11a, 11b) comprises at least one curved inner face (13).

6. Use according to one of the preceding claims, **characterized in that** the hanging device (3) of the cowling (1) pivots between the rest position and the hanging position by an angle (α) of at least 110°.

7. Use according to one of the preceding claims, **characterised in that** the cowling (1) comprises at least one support strut (5) secured to the cowling body (2) and carrying a handle (4).

8. Use according to one of the preceding claims, **characterised in that** the gas container (10) is selected from among gas cylinders.

9. Use according to one of the preceding claims, **characterised in that** the gas container (10) is a gas cylinder of a size of between 10 and 150cm, and containing 0.5 to 20 litres (water-equivalent volume).
